(19) **Europäisches Patentamt / European Patent Office / Office européen des brevets**

(11) **EP 2 821 039 B1**

(12) **EUROPEAN PATENT SPECIFICATION**

(45) Date of publication and mention of the grant of the patent:
**10.07.2019 Bulletin 2019/28**

(21) Application number: **13755348.3**

(22) Date of filing: **18.02.2013**

(51) Int Cl.:
*A61F 13/15* (2006.01)      *A61F 13/00* (2006.01)
*A61F 13/472* (2006.01)     *A61F 13/511* (2006.01)
*A61L 15/50* (2006.01)      *A61L 15/24* (2006.01)

(86) International application number:
**PCT/JP2013/053879**

(87) International publication number:
**WO 2013/129163 (06.09.2013 Gazette 2013/36)**

(54) **ABSORBENT ARTICLE**

SAUGFÄHIGER ARTIKEL

ARTICLE ABSORBANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.02.2012 JP 2012043969**

(43) Date of publication of application:
**07.01.2015 Bulletin 2015/02**

(73) Proprietor: **Unicharm Corporation Ehime 799-0111 (JP)**

(72) Inventors:
• **WADA, Ichiro**
  **Kanonji-shi**
  **Kagawa 769-1602 (JP)**

• **NAKASHITA, Masashi**
  **Kanonji-shi**
  **Kagawa 769-1602 (JP)**

(74) Representative: **Dolleymores**
   **9 Rickmansworth Road**
   **Watford, Hertfordshire WD18 0JU (GB)**

(56) References cited:
   **WO-A1-03/028776      JP-A- 2009 247 418**
   **JP-A- 2010 035 800   JP-A- 2011 510 785**
   **JP-A- 2011 510 801   US-A1- 2009 209 930**
   **US-A1- 2011 070 277**

**Description**

Technical Field

The present disclosure relates to use of an agent in a liquid-permeable top sheet in an absorbent article for modifying blood.

Background Art

As the basic performance of absorbent articles such as sanitary napkins and panty liners has continued to improve with technological development over many years, leakage after absorption of excreta such as menstrual blood has become a less frequent occurrence than in the past, and research is currently ongoing with the aim of achieving even higher performance, including a feel similar to underwear, and smoothness of the top sheet even after absorption of excreta such as menstrual blood.

Menstrual blood during menstruation, in particular, can also contain components of the endometrium which are highly viscous, and the top sheet preferably remains smooth and stick-free even after absorption of such highly viscous menstrual blood. Highly viscous menstrual blood usually remains on the top sheet in the form of masses, generally leaving the user with a visually unpleasant image, and therefore from this viewpoint as well it is preferred for no highly viscous menstrual blood to remain on the top sheet.

In recent years, absorbent articles having indented shapes such as ridge-furrow shapes formed on the skin contact surface of the top sheet, have been marketed as absorbent articles that maintain smooth top sheets even after absorption of excreta such as menstrual blood. Other conventional absorbent articles that have flat top sheets, i.e. lacking indented shapes on the top sheet, are still being marketed principally in developing countries, and such absorbent articles are also preferred to have smooth top sheets even after absorption of excreta such as menstrual blood.

For example, PTL 1 discloses an absorbent article having a polypropylene glycol material-containing lotion composition situated on the inner surface of the top sheet (the clothing side surface), the inner surface of the back sheet (the body side surface), and on the base material between the inner surface of the top sheet and the inner surface of the back sheet.

Also, PTL 2 discloses an absorbent article wherein a polypropylene glycol material-containing lotion composition is applied on the outer surface of the top sheet (body side surface).

Citation List

Patent Literature

PTL 1 Japanese Unexamined Patent Publication No. 2010-518918
PTL 2 Japanese Unexamined Patent Publication No. 2011-510801

US 2009/0209930 A1 discloses an absorbent article for wearing in an undergarment. The absorbent article can include a nonwoven. The nonwoven can have a nonwoven body facing surface. The nonwoven can have a main body portion and pair of spaced apart flaps associated with the main body portion. Part of the main body portion can have a hydrophilic zone that is more hydrophilic than a portion of the flaps. The absorbent article can have film having a film garment facing surface, wherein at least part of the film garment facing surface faces the nonwoven body facing surface.

US 2011/0070277 A1 relates to a lotion composition applied on the body facing surface of an absorbent article and improves the ease of removal of feces or menses after an absorbent article comprising the lotion composition has been used and removed from the wearer.

WO 03/028776 A1 discloses a catamenial device, the device having a topsheet having a body facing surface, wherein the topsheet has a level of hydrophobicity. A lotion composition is applied to at least a portion of at least the body facing surface of the topsheet, the lotion composition having a level of hydrophobicity equal or greater than that of said topsheet.

Summary of Invention

Technical Problem

However, the inventions described in PTL 1 and 2 do not discuss the types and shapes of the top sheets.

It is therefore an object of the present disclosure to provide an absorbent article lacking conventional indented shapes formed on the top sheet, and without a sticky feel on the top sheet and maintaining a smooth top sheet, even after highly viscous menstrual blood has been absorbed.

Solution to Problems

As a result of diligent research directed toward solving the problems described above, the present inventors have discovered the present invention which provides the use of independent claim 1. The dependent claims specify preferred but optional features.

Advantageous Effects of Invention

The use of an agent of the disclosure in a liquid-permeable top sheet in an absorbent article has no sticky feel on the top sheet and maintains a smooth top sheet, even after highly viscous menstrual blood has been absorbed.

Brief Description of Drawings

Fig. 1 is a front view of a sanitary napkin, as an example of an absorbent article.

Fig. 2 is schematic view showing the state of absorption of menstrual blood by the absorbent article of the present disclosure.

Fig. 3 is a schematic view showing the state of absorption of menstrual blood by an absorbent article having a surfactant coated on the skin contact surface of the top sheet.

Description of Embodiments

The absorbent article of the present disclosure will now be explained in detail.

[Blood modifying agent]

The blood modifying agent in the absorbent article of the present disclosure is a polyoxypropylene glycol-based compound. The blood modifying agent is not particularly restricted so long as it is a polyoxypropylene glycol-based compound, i.e. a compound containing a polyoxypropylene glycol backbone, but preferred examples are those with the following structures.

The blood modifying agent is preferably selected from the group consisting of the following (A) to (E):

(A) polyoxypropylene glycol,

(B) a mono or diether of (B1) polyoxypropylene glycol and (B2) a compound having a hydrocarbon moiety and one hydroxyl group substituting for a hydrogen of the hydrocarbon moiety,

(C) a mono or diester of (C1) polyoxypropylene glycol and (C2) a compound having a hydrocarbon moiety and one carboxyl group substituting for a hydrogen of the hydrocarbon moiety,

(D) a compound produced by addition of (D2) one or more propylene oxides to (D1) a compound having a hydrocarbon moiety and 2-6 hydroxyl groups substituting for hydrogens of the hydrocarbon moiety or dehydrating condensate thereof, and optional esterification with (D3) a compound having a hydrocarbon moiety and one carboxyl group substituting for a hydrogen of the hydrocarbon moiety, or phosphoric acid, and

(E) a compound produced by addition of (E2) one or more propylene oxides to (E1) an alkylamide, alkylamine or methylglucose, and optional esterification with (E3) a compound having a hydrocarbon moiety and one carboxyl group substituting for a hydrogen of the hydrocarbon moiety, or phosphoric acid.

As used herein, the term "hydrocarbon" in "hydrocarbon moiety" refers to a compound composed of carbon and hydrogen, and it includes chain hydrocarbons, such as paraffinic hydrocarbons (containing no double bond or triple bond, also referred to as "alkanes"), olefin-based hydrocarbons (containing one double bond, also referred to as "alkenes"), acetylene-based hydrocarbons (containing one triple bond, also referred to as "alkynes"), and hydrocarbons comprising two or more bonds selected from the group consisting of double bonds or triple bonds, and cyclic hydrocarbons such as aromatic hydrocarbons and alicyclic hydrocarbons.

Preferred as such hydrocarbons are chain hydrocarbons and alicyclic hydrocarbons, with chain hydrocarbons being more preferred, paraffinic hydrocarbons, olefin-based hydrocarbons and hydrocarbons with two or more double bonds (containing no triple bond) being more preferred, and paraffinic hydrocarbons being even more preferred.

Chain hydrocarbons include chain hydrocarbons and branched-chain hydrocarbons.

The blood modifying agent according to (A) to (E) will now be explained in detail.

[(A) Polyoxypropylene glycol]

The (A) polyoxypropylene glycol (hereunder, "compound (A)") is represented by the following formula (1) .

$$HO\text{-}(C_3H_6O)_n\text{-}H \qquad (1)$$

The value of n is preferably between about 4 to 50, more preferably between about 5 to 40, and even more preferably between about 10 to 30.

Examples of commercial products for compound (A) include the following (all by NOF Corp.).

- UNIOL D-250
  Polyoxypropylene glycol, weight-average molecular weight: approximately 250
- UNIOL D-400
  Polyoxypropylene glycol, weight-average molecular weight: approximately 400
- UNIOL D-700
  Polyoxypropylene glycol, weight-average molecular weight: approximately 700
- UNIOL D-1000
  Polyoxypropylene glycol, weight-average molecular weight: approximately 1,000
- UNIOL D-1200
  Polypropylene glycol, weight-average molecular weight: approximately 1,200
- UNIOL D-2000
  Polypropylene glycol, weight-average molecular weight: approximately 2,030
- UNIOL D-3000
  Polypropylene glycol, weight-average molecular weight: approximately 3,000
- UNIOL D-4000
  Polypropylene glycol, weight-average molecular weight: approximately 4,000

[(B) Ether of (B1) polyoxypropylene glycol and (B2) compound having a hydrocarbon moiety and one hydroxyl group substituting for a hydrogen of the hydrocarbon moiety]

In the (B) mono or diether of (B1) a polyoxypropylene glycol and (B2) a compound having a hydrocarbon moiety and one hydroxyl group substituting for a hydrogen of the hydrocarbon moiety (hereunder also referred to as "compound (B)"), the (B1) polyoxypropylene glycol may be any of those listed for compound (A).

The (B2) compound having a hydrocarbon moiety and one hydroxyl group substituting for a hydrogen of the hydrocarbon moiety may be a chain hydrocarbon monool, such as an alkane monool, such as a $C_1$-$C_{20}$ aliphatic monohydric alcohol.

Examples of $C_1$-$C_{20}$ aliphatic monohydric alcohols include $C_1$-$C_{20}$ saturated aliphatic monohydric alcohols and $C_1$-$C_{20}$ unsaturated aliphatic monohydric alcohols.

Examples of $C_1$-$C_{20}$ saturated aliphatic monohydric alcohols include methyl alcohol ($C_1$) ($C_1$ representing the number of carbon atoms, same hereunder), ethyl alcohol ($C_2$), propyl alcohol ($C_3$) and isomers thereof, including isopropyl alcohol ($C_3$), butyl alcohol ($C_4$) and isomers thereof, including sec-butyl alcohol ($C_4$) and tert-butyl alcohol ($C_4$), pentyl alcohol ($C_5$), hexyl alcohol ($C_6$), heptyl alcohol ($C_7$), octyl alcohol ($C_8$) and isomers thereof, including 2-ethylhexyl alcohol ($C_8$), nonyl alcohol ($C_9$), decyl alcohol ($C_{10}$), dodecyl alcohol ($C_{12}$), tetradecyl alcohol ($C_{14}$), hexadecyl alcohol ($C_{16}$), heptadecyl alcohol ($C_{17}$), octadecyl alcohol ($C_{18}$) and eicosyl alcohol ($C_{20}$), as well as their isomers other than those mentioned.

Among $C_1$-$C_{20}$ unsaturated aliphatic monohydric alcohols there may be mentioned those wherein one C-C single bond of a saturated aliphatic monohydric alcohol mentioned above is replaced with a C=C double bond, such as oleyl alcohol, and for example, such alcohols are commercially available by New Japan Chemical Co., Ltd. as the RIKACOL Series and UNJECOL Series.

Examples of commercial products for compound (B) include the following (all by NOF Corp.). The trade name is listed first, followed by the name according to "International Nomenclature of Cosmetic Ingredients".

- UNILUBE MB-2, PPG-4 butyl ether
- UNILUBE MB-7, PPG-12 butyl ether
- UNILUBE MB-11, PPG-17 butyl ether
- UNILUBE MB-14, PPG-20 butyl ether
- UNILUBE MB-19, PPG-24 butyl ether

- UNILUBE MB-38, PPG-33 butyl ether
- UNILUBE MB-370, PPG-40 butyl ether
- UNILUBE MB-700, PPG-52 butyl ether
- UNISAFE MM-15K, PPG-3 myristyl ether
- UNILUBE MP-60K, PPG-10 cetyl ether
- UNILUBE MS-65K, PPG-11 stearyl ether

- UNILUBE MS-70K, PPG-15 stearyl ether
- UNILUBE MLA-202P, PPG-2 lanolyl ether
- UNILUBE MLA-205P, PPG-5 lanolyl ether
- UNILUBE MLA-210P, PPG-10 lanolyl ether
- UNILUBE MLA-220P, PPG-20 lanolyl ether

[(C) Mono or diester of (C1) polyoxypropylene glycol and (C2) compound having a hydrocarbon moiety and one carboxyl group substituting for a hydrogen of the hydrocarbon moiety]

In the (C) mono or diester of (C1) a polyoxypropylene glycol and (C2) a compound having a hydrocarbon moiety and one carboxyl group substituting for a hydrogen of the hydrocarbon moiety (hereunder also referred to as "compound (C)"), the (C1) polyoxypropylene glycol may be any of those listed for compound (A).

The (C2) compound having a hydrocarbon moiety and one carboxyl group substituting for a hydrogen of the hydrocarbon moiety (hereunder also referred to as "compound (C2)") may be a $C_2$-$C_{30}$ fatty acid, such as a $C_2$-$C_{30}$ saturated fatty acid or a $C_2$-$C_{30}$ unsaturated fatty acid.

Examples of $C_2$-$C_{30}$ saturated fatty acids include acetic acid ($C_2$) (the subscript numeral of "$C_2$" representing the number of carbon atoms, same hereunder), propanoic acid ($C_3$), butanoic acid ($C_4$) and isomers thereof, such as 2-methylpropanoic acid ($C_4$), pentanoic acid ($C_5$) and isomers thereof such as 2-methylbutanoic acid ($C_5$) and 2,2-dimethylpropanoic acid ($C_5$), hexanoic acid ($C_6$), heptanoic acid ($C_7$), octanoic acid ($C_8$) and isomers thereof, such as 2-ethylhexanoic acid ($C_8$), nonanoic acid ($C_9$), decanoic acid ($C_{10}$), dodecanoic acid ($C_{12}$), tetradecanoic acid ($C_{14}$), hexadecanoic acid ($C_{16}$), heptadecanoic acid ($C_{17}$), octadecanoic acid ($C_{18}$), eicosanoic acid ($C_{20}$), docosanoic acid ($C_{22}$), tetracosanoic acid ($C_{24}$), hexacosanoic acid ($C_{26}$), octacosanoic acid ($C_{28}$), triacontanoic acid ($C_{30}$) and the like, as well as isomers of the above that are not mentioned.

Examples of $C_2$-$C_{30}$ unsaturated fatty acids include monounsaturated fatty acids such as crotonic acid ($C_4$), myristoleic acid ($C_{14}$), palmitoleic acid ($C_{16}$), oleic acid ($C_{18}$), elaidic acid ($C_{18}$), vaccenic acid ($C_{18}$), gadoleic acid ($C_{20}$) and eicosenoic acid ($C_{20}$), di-unsaturated fatty acids including linolic acid ($C_{18}$) and eicosadienoic acid ($C_{20}$), tri-unsaturated fatty acids including linolenic acids, such as $\alpha$-linolenic acid ($C_{18}$) and $\gamma$-linolenic acid ($C_{18}$), pinolenic acid ($C_{18}$), eleostearic acids, such as $\alpha$-eleostearic acid ($C_{18}$) and $\beta$-eleostearic acid ($C_{18}$), Mead acid ($C_{20}$), dihomo-$\gamma$-linolenic acid ($C_{20}$) and eicosatrienoic acid ($C_{20}$), tetra-unsaturated fatty acids including stearidonic acid ($C_{20}$), arachidonic acid ($C_{20}$) and eicosatetraenoic acid ($C_{20}$), penta-unsaturated fatty acids including bosseopentaenoic acid ($C_{18}$) and eicosapentaenoic acid ($C_{20}$), and partial hydrogen adducts of the foregoing.

Examples of commercial products for compound (C) include the following (all by NOF Corp.). The trade name is listed first, followed by the name according to "International Nomenclature of Cosmetic Ingredients".

- UNILUBE 0-1500, PPG-26 oleate
- UNILUBE 0-2100, PPG-36 oleate
- UNILUBE CF-320, PPG-5.5 castorate

[(D) Compound produced by addition of (D2) one or more propylene oxides to (D1) compound having a hydrocarbon moiety and 2-6 hydroxyl groups substituting for hydrogens of the hydrocarbon moiety or dehydrating condensate thereof, and optional esterification with (D3) compound having a hydrocarbon moiety and one carboxyl group substituting for a hydrogen of the hydrocarbon moiety, or (D4) phosphoric acid]

The (D) compound produced by addition of (D2) one or more propylene oxides to (D1) a compound having a hydrocarbon moiety and 2-6 hydroxyl groups substituting for hydrogens of the hydrocarbon moiety or dehydrating condensate thereof, and optional esterification with (D3) a compound having a hydrocarbon moiety and one carboxyl group substituting for a hydrogen of the hydrocarbon moiety, or (D4) phosphoric acid (hereunder also referred to as "compound (D)" will now be described.

The (D1) compound having a hydrocarbon moiety and 2-6 hydroxyl groups substituting for hydrogens of the hydrocarbon moiety (hereunder also referred to as "compound (D1)") may be a compound having a chain hydrocarbon moiety and 2-6 hydroxyl groups substituting for hydrogens of the chain hydrocarbon moiety.

Examples of compounds having a chain hydrocarbon moiety and 2-6 hydroxyl groups substituting for hydrogens of the chain hydrocarbon moiety include chain hydrocarbon hexaols including alkane hexaols such as sorbitol (glucitol), galactitol and mannitol, chain hydrocarbon pentaols including alkane pentaols such as xylitol and ribitol, chain hydrocarbon tetraols including alkane tetraols such as pentaerythritol, chain hydrocarbon triols including alkane triols such as glycerin, and chain hydrocarbon diols including alkane diols such as glycol.

Compound (D1) is more preferably selected from the group consisting of sorbitol, pentaerythritol, glycerin, trimethylolpropane and ethylene glycol, and any desired combinations thereof.

For compound (D1), the sorbitol (glucitol), galactitol, mannitol, xylitol, ribitol, pentaerythritol and glycerin are naturally produced alditols, and they are therefore preferred from the viewpoint of safety.

The (D2) one or more propylene oxides (hereunder also referred to as "compound (D2)") may be 1,2-propylene oxide or 1,3-propylene oxide.

Compound (D) may be derived by addition of preferably about 1 to about 60 mol, more preferably about 2 to about 50 mol and even more preferably about 3 to about 40 mol of compound (D2) to 1 mol of compound (D1).

The (D3) compound having a hydrocarbon moiety and one carboxyl group substituting for a hydrogen of the hydrocarbon moiety is an optional component and may be any of those listed as compound (C2) for compound (C), and for example, it may be a $C_2$-$C_{30}$ fatty acid, such as a $C_2$-$C_{30}$ saturated fatty acid or a $C_2$-$C_{30}$ unsaturated fatty acid.

The (D4) phosphoric acid is also an optional component.

Examples of commercial products for compound (D) include the following (all by NOF Corp.). The trade name is listed first, followed by the name according to "International Nomenclature of Cosmetic Ingredients".

- UNIOL TG-330, PPG-6 glyceryl ether
- UNIOL SGP-65, PPG-8 glyceryl ether
- UNIOL TG-700, PPG-10 glyceryl ether
- UNIOL TG-1000, PPG-16 glyceryl ether

- UNILUBE DGP-700, PPG-9 diglyceryl ether
- UNILUBE DGP-950, PPG-14 diglyceryl ether
- UNIOL HS-1600D, PPG-25 sorbitol
- UNIOL HS-2000D, PPG-33 sorbitol

[(E) Compound produced by addition of (E2) one or more propylene oxides to (E1) aliphatic amide, aliphatic amine or alkylglucose, and optional esterification with (E3) compound having a hydrocarbon moiety and one carboxyl group substituting for a hydrogen of the hydrocarbon moiety, or (E4) phosphoric acid]

The (E) compound produced by addition of (E2) one or more propylene oxides to (E1) an aliphatic amide, aliphatic amine or alkylglucose, and optional esterification with (E3) a compound having a hydrocarbon moiety and one carboxyl group substituting for a hydrogen of the hydrocarbon moiety, or (E4) phosphoric acid (hereunder also referred to as "compound (E)") will now be described.

In compound (E), the aliphatic amide has a group represented by the following formula (2):

$$R^1\text{-C(=O)NH}_2 \qquad \text{Formula (2)}$$

(wherein $R^1$ is a chain hydrocarbon group).

Examples for the aliphatic amide include $C_1$-$C_{20}$ aliphatic amides such as $C_1$-$C_{20}$ saturated aliphatic amides, including $C_1$-$C_{20}$ alkylamides.

$C_1$-$C_{20}$ alkyl groups include groups represented by the following formula (2'):

$$R^2\text{-}C(=O)NH_2 \qquad \text{Formula (2')}$$

(wherein $R^2$ is a C1-20 alkyl group).

Examples for $R^2$ include methyl ($C_1$) (where $C_1$ indicates the number of carbon atoms, same hereunder), ethyl ($C_2$), propyl ($C_3$) and isomers thereof, such as isopropyl ($C_3$), butyl ($C_4$) and isomers thereof, such as sec-butyl ($C_4$) and tert-butyl ($C_4$), pentyl ($C_5$), hexyl ($C_6$), heptyl ($C_7$), octyl ($C_8$) and isomers thereof, such as 2-ethylhexyl ($C_8$), nonyl ($C_9$), decyl ($C_{10}$), dodecyl ($C_{12}$), tetradecyl ($C_{14}$), hexadecyl ($C_{16}$), heptadecyl ($C_{17}$), octadecyl ($C_{18}$) and eicosyl ($C_{20}$), and isomers of the foregoing other than those mentioned above.

For compound (E), the aliphatic amine has a group represented by the following formula (3):

$$R^3\text{-}NH_2 \qquad \text{Formula (3)}$$

(wherein $R^3$ is a chain hydrocarbon group).

Examples for the aliphatic amine include $C_1$-$C_{20}$ aliphatic amines such as $C_1$-$C_{20}$ saturated aliphatic amines, including $C_1$-$C_{20}$ alkylamines.

$C_1$-$C_{20}$ alkylamines include groups represented by the following formula (3'):

$$R^4\text{-}NH_2 \qquad \text{Formula (3')}$$

(wherein $R^4$ is a C1-20 alkyl group).

Examples for $R^4$ include methyl ($C_1$) (where $C_1$ indicates the number of carbon atoms, same hereunder), ethyl ($C_2$), propyl ($C_3$) and isomers thereof, such as isopropyl ($C_3$), butyl ($C_4$) and isomers thereof, such as sec-butyl ($C_4$) and tert-butyl ($C_4$), pentyl ($C_5$), hexyl ($C_6$), heptyl ($C_7$), octyl ($C_8$) and isomers thereof, such as 2-ethylhexyl ($C_8$), nonyl ($C_9$), decyl ($C_{10}$), dodecyl ($C_{12}$), tetradecyl ($C_{14}$), hexadecyl ($C_{16}$), heptadecyl ($C_{17}$), octadecyl ($C_{18}$) and eicosyl ($C_{20}$), and isomers of the foregoing other than those mentioned above.

Examples of alkylglucoses include methylglucose, ethylglucose and propylglucose.

The (E2) one or more propylene oxides may be the same as for compound (D2).

The (E3) compound having a hydrocarbon moiety and one carboxyl group substituting for a hydrogen of the hydrocarbon moiety is an optional component and may be any of those listed as compound (C2) for compound (C), and for example, it may be a $C_2$-$C_{30}$ fatty acid, such as a $C_2$-$C_{30}$ saturated fatty acid or a $C_2$-$C_{30}$ unsaturated fatty acid.

The (E4) phosphoric acid is also an optional component.

Even more preferably, the blood modifying agent is selected from the group consisting of PPG-4 butyl ether, PPG-12 butyl ether, PPG-17 butyl ether, PPG-20 butyl ether, PPG-24 butyl ether, PPG-33 butyl ether, PPG-40 butyl ether, PPG-52 butyl ether, PPG-3 myristyl ether, PPG-10 cetyl ether, PPG-11 stearyl ether, PPG-15 stearyl ether, PPG-2 lanolyl ether, PPG-5 lanolyl ether, PPG-10 lanolyl ether, PPG-20 lanolyl ether, PPG-26 oleate, PPG-36 oleate, PPG-5.5 castorate, PPG-6 glyceryl ether, PPG-8 glyceryl ether, PPG-10 glyceryl ether, PPG-16 glyceryl ether, PPG-9 diglyceryl ether, PPG-14 diglyceryl ether, PPG-25 sorbitol and PPG-33 sorbitol, as well as any desired combinations of the foregoing.

Other examples of blood modifying agents include PPG-30 cetyl ether, PPG-15 isohexadecyl ether, PPG-4 lauryl ether, PPG-20 distearate, PPG-12 dilaurate, PPG-15 dicocoate, PPG-10 cetyl phosphate, PPG-9 laurate, PPG-8 dioctate, PPG-15 stearate, PPG-8 diethyl hexylate, PPG-10 glyceryl stearate, PPG-2 cocamide, PPG-10 tallow amine, PPG-10 oleamide, PPG-5 sucrose cocoate, PPG-20 methylglucose ether distearate, PPG-20 methylglucose ether acetate, PPG-20 sorbitan tristearate, PPG-20 methylglucose ether distearate, PPG-15 stearyl ether benzoate, PPG-10 sorbitan monostearate, PPG-10 hydrogenated castor oil, PPG-10 cetyl ether phosphate, PPG-10 dinonyl phenolate, PPG-7 lauryl ether, PPG-5 lanolin wax ether, PPG-5 lanolin wax, PPG-4 jojoba alcohol ether, PPG-3 myristyl ether propionate, PPG-3 benzyl ether myristate, PPG-3 hydrogenated castor oil, PPG-3-hydroxyethyl soyamide, PPG-2 lanolin alcohol ether and PPG-1 coconut fatty acid isopropanolamide.

The blood modifying agent has a water holding percentage of about 1.5 to about 40.0 mass%, preferably it has a water holding percentage of about 1.6 to about 30.0 mass%, and more preferably it has a water holding percentage of about 1.8 to about 20.0 mass%.

As used herein, "water holding percentage" means the percentage of water that can be held by a substance, and it may be measured in the following manner.

(1) A test tube, a rubber stopper, the substance to be measured and deionized water are allowed to stand for a day and a night in a thermostatic chamber at 40°C.

(2) Into the 20 mL test tube in the thermostatic chamber there are charged 5.0 g of the substance to be measured and 5.0 g of deionized water.

(3) The mouth of the test tube is sealed with the rubber stopper in the thermostatic chamber, and it is rotated once and allowed to stand for 5 minutes.

(4) A 3.0 g portion of the layer of the substance to be measured (usually the upper layer) is sampled into a glass dish with a diameter of 90 mm (weight: $W_0$), in the thermostatic chamber.

(5) The dish is heated at 105°C for 3 hours in an oven to evaporate off the moisture, and the weight of each dish is measured (weight: $W_1$).

(6) The water holding percentage is calculated by the following formula.

$$\text{Water holding percentage (\%)} = 100 \times (W_0 - W_1)/3.0$$

The measurement is conducted three times, and the average value is recorded.

If the water holding percentage is low, affinity between the blood modifying agent and menstrual blood is reduced, and menstrual blood that has reached the skin contact surface of the top sheet does not easily spread thinly across the skin contact surface of the top sheet, which tends to slow permeation of menstrual blood into the top sheet, and as a result menstrual blood tends to be poorly drawn through the absorbent body. If the water holding percentage is high, on the other hand, the affinity between menstrual blood and the blood modifying agent will become very high, similar to a surfactant, and menstrual blood will easily remain on the skin contact surface of the top sheet, resulting in a tendency toward red coloration of the skin contact surface of the top sheet.

The water holding percentage tends to be a larger value with a) a smaller molecular weight of the blood modifying agent, and b) a higher percentage of polar groups such as carbonyl bonds (-CO-), ether bonds (-O-), carboxyl groups (-COOH) and hydroxyl groups (-OH). This is because the blood modifying agent has greater hydrophilicity.

The weight-average molecular weight of the blood modifying agent is not particularly restricted, but it is preferably a weight-average molecular weight of less than about 3,000, more preferably a weight-average molecular weight of less than about 2,000 and even more preferably a weight-average molecular weight of less than about 1,000. An increased weight-average molecular weight will tend to produce tack in the blood modifying agent itself, creating a feeling of unpleasantness for the wearer. Also, a high weight-average molecular weight will tend to result in high viscosity of the blood modifying agent, and it will be difficult to lower the viscosity of the blood modifying agent by heating, to a viscosity suitable for coating. As a result, it will sometimes be necessary to dilute the blood modifying agent with a solvent.

The blood modifying agent preferably has a weight-average molecular weight of about 100 or greater, and more preferably it has a weight-average molecular weight of about 200 or greater. This is because if the weight-average molecular weight is low, the vapor pressure may be increased, gasification may occur during storage and the amount of blood modifying agent may be reduced, often leading to problems such as odor during wear.

As used herein, "weight-average molecular weight" includes the concept of a polydisperse compound (for example, a compound produced by stepwise polymerization, an ester formed from a plurality of fatty acids and a plurality of aliphatic monohydric alcohols), and a simple compound (for example, an ester formed from one fatty acid and one aliphatic monohydric alcohol), and in a system comprising $N_1$ molecules with molecular weight $M_i$ (i = 1, or i = 1, 2 ...), it refers to $M_w$ determined by the following formula.

$$M_w = \Sigma N_i M_i{}^2 / \Sigma N_i M_i$$

The weight-average molecular weights used throughout the present specification are the values measured by gel permeation chromatography (GPC), based on polystyrene.

The GPC measuring conditions may be the following, for example.

Device: Lachrom Elite high-speed liquid chromatogram by Hitachi High-Technologies Corp.

Columns: SHODEX KF-801, KF-803 and KF-804, by Showa Denko K.K.

Eluent: THF

Flow rate: 1.0 mL/min

Driving volume: 100 $\mu$L

Detection: RI (differential refractometer)

The weight-average molecular weights listed in the examples of the present specification were measured under the conditions described below.

The blood modifying agent preferably has a melting point of no higher than 45°C. If the blood modifying agent has a melting point of higher than 45°C, the blood modifying agent will include solid matter at temperatures near the range between body temperature and 40°C, and it may fail to act on menstrual blood.

As used herein, the term "melting point" refers to the peak top temperature for the endothermic peak during conversion from solid to liquid, upon measurement with a differential scanning calorimetry analyzer at a temperature-elevating rate of 10°C/min. The melting point may be measured using a Model DSC-60 DSC measuring apparatus by Shimadzu Corp., for example.

The blood modifying agent may be either a liquid or solid at room temperature (approximately 25°C), or in other words, the melting point may be either about 25°C or higher or below about 25°C, and for example, it may have a melting point of about -5°C or about -20°C.

The blood modifying agent does not have a lower limit for the melting point, but the vapor pressure is preferably low. The vapor pressure of the blood modifying agent is preferably about 0-200 Pa, more preferably about 0-100 Pa, even more preferably about 0-10 Pa, even more preferably about 0-1 Pa, and even more preferably about 0.0-0.1 Pa at 25°C (1 atmosphere).

Considering that the absorbent article of this disclosure is used in contact with the human body, the vapor pressure is preferably about 0-700 Pa, more preferably about 0-100 Pa, even more preferably about 0-10 Pa, even more preferably about 0-1 Pa, and even more preferably 0.0-0.1 Pa, at 40°C (1 atmosphere). If the vapor pressure is high, gasification may occur during storage and the amount of blood modifying agent may be reduced, and as a consequence problems, such as odor during wear, may be created.

The melting point of the blood modifying agent may be selected depending on the weather or duration of wear. For example, in regions with a mean atmospheric temperature of no higher than about 10°C, using a blood modifying agent with a melting point of no higher than about 10°C may help the blood modifying agent function after excretion of menstrual blood, even if it has been cooled by the ambient temperature.

Also, when the absorbent article is used for a prolonged period of time, the melting point of the blood modifying agent is preferably at the high end of the range of no higher than about 45°C. This is because the blood modifying agent is not easily affected by sweat or friction during wearing, and will not easily become biased even during prolonged wearing.

[Liquid-permeable top sheet]

The liquid-permeable top sheet has a thickness of between about 0.10 mm and about 0.30 mm and preferably a thickness of between about 0.15 mm and about 0.30 mm in the excretory opening contact region. In the absorbent article of the present disclosure, a blood modifying agent with high affinity for menstrual blood is present in the skin contact surface of the top sheet, and therefore menstrual blood that has reached the skin contact surface of the top sheet spreads out thinly, the contact area between the menstrual blood and the top sheet is increased and the menstrual blood migrates into the top sheet, after which the menstrual blood inside the top sheet is rapidly drawn into the absorbent body. If the thickness of the top sheet exceeds about 0.3 mm, however, menstrual blood inside the top sheet will not be easily drawn into the absorbent body.

The material of the liquid-permeable top sheet in the absorbent article of the present disclosure is a nonwoven fabric.

The thickness of each material including the top sheet can be measured using a laser displacement meter, such as an LJ-G Series high precision two-dimensional laser displacement gauge (Model: LJ-G030) by Keyence Corp.

The absorbent article of the present disclosure has a basis weight of between about 5 and about 30 $g/m^2$, preferably a basis weight of between about 10 and about 25 $g/m^2$ and more preferably a basis weight of between about 15 and about 20 $g/m^2$, in the excretory opening contact region. If the basis weight is lower than 5 $g/m^2$, the strength as a top sheet will tend to be insufficient, while if the basis weight is higher than 30 $g/m^2$, the bulk of the top sheet will be increased and the absorbent body will tend to draw in menstrual blood with difficulty. The top sheet is a nonwoven fabric and the number of fibers per unit area will be high, and the blood modifying agent that has high affinity for menstrual blood will tend to hold the menstrual blood between the fibers of the nonwoven fabric.

The liquid-permeable top sheet preferably has a water pressure resistance of between about 50 mm and about 100 mm, and more preferably a water pressure resistance of between about 55 mm and about 65 mm, in the excretory opening contact region. If the water pressure resistance is lower than about 50 mm the strength as a top sheet will tend to be insufficient, and if the water pressure resistance is higher than about 100 mm the top sheet will tend to be hard, resulting in an inferior feel.

The water pressure resistance is the water pressure resistance measured according to JIS L 1092:2009, Moisture Resistance Test Method for Fiber Products, "7.1 Water penetration test (hydrostatic pressure method)", "Method 7.1.1A (low hydraulic pressure test)".

The liquid-permeable top sheet of the absorbent article of the present disclosure is a nonwoven fabric being a spunbond nonwoven fabric or a spunbond/meltblown/spunbond nonwoven (SMS) fabric.

According to the absorbent article of the present disclosure the liquid-permeable top sheet is a spunbond nonwoven fabric or a spunbond/meltblown/spunbond nonwoven fabric (SMS nonwoven fabric), having a three-layer structure. Since spunbond nonwoven fabrics and SMS nonwoven fabrics are produced worldwide as hygienic general purpose nonwoven fabrics, they are available at relatively low cost and are widely used as top sheets for absorbent articles even in developing countries. The present disclosure is therefore highly useful since it can provide top sheets formed from spunbond nonwoven fabrics or SMS nonwoven fabrics with a non-sticky and a smooth feel.

The following description concerns the reason for which the absorbent article of the present disclosure has no sticky feel on the top sheet and maintains a smooth top sheet, even after highly viscous menstrual blood has been absorbed.

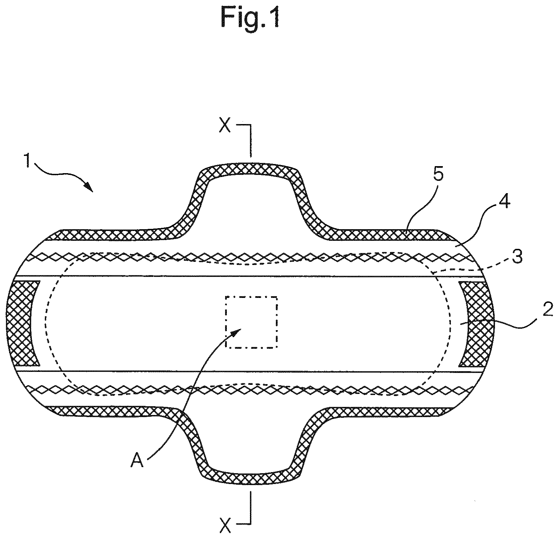

Fig. 1 is a front view of a sanitary napkin, as viewed from the skin contact side. The sanitary napkin 1 shown in Fig. 1 has its forward direction facing left in the drawing. The sanitary napkin 1 shown in Fig. 1 has a liquid-permeable top sheet 2, an absorbent body 3, and a liquid-impermeable back sheet (not shown). The sanitary napkin 1 in Fig. 1 is also shown as having a side sheet 4 and stamped sections 5.

The side sheet 4 and stamped section 5 are shown for the absorbent article illustrated in Fig. 1, but another absorbent article of the present disclosure is an absorbent article without a side sheet and stamped sections.

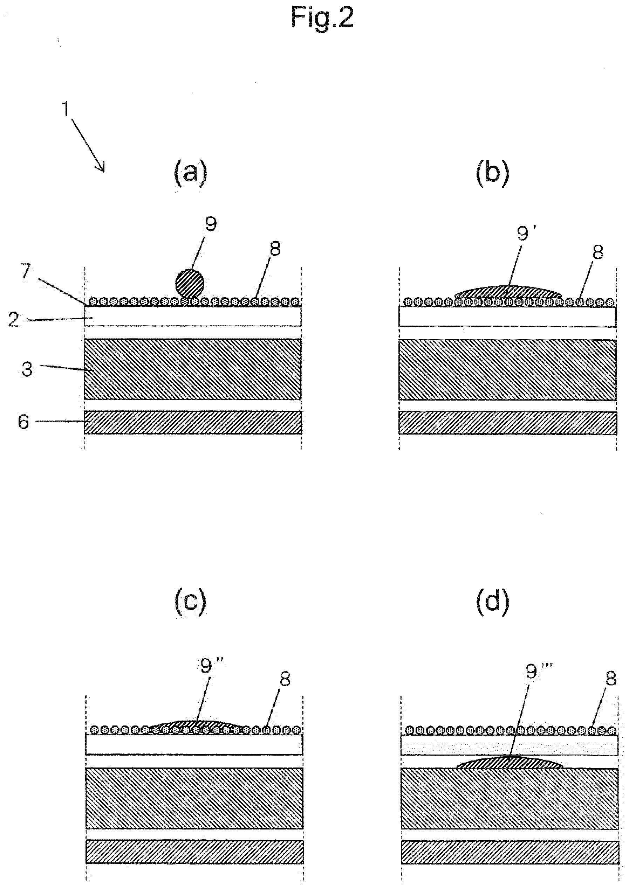

Fig. 2 is schematic view showing the state of absorption of menstrual blood by the absorbent article of the present disclosure. Fig. 2 is a cross-sectional view of section A of the sanitary napkin 1 shown in Fig. 1, along X-X. The sanitary napkin 1 shown in Fig. 2 comprises a liquid-permeable top sheet 2, a liquid-impermeable back sheet 6, and an absorbent body 3 between the liquid-permeable top sheet 2 and liquid-impermeable back sheet 6. In Fig. 2, the top sheet 2 has a thickness of no greater than about 0.3 mm, and a blood modifying agent 8 is coated on the skin contact surface 7 of the top sheet 2. In Fig. 2, the blood modifying agent 8 is shown as droplets on the skin contact surface 7 of the top sheet 2 for convenience, but according to the absorbent article of the present disclosure, the form and distribution of the blood modifying agent is not limited to that shown in the drawing.

As shown in Fig. 2(a), highly viscous menstrual blood 9 reaches the skin contact surface 7 of the top sheet 2 and contacts with the blood modifying agent 8. The blood modifying agent 8 with the prescribed structure has high affinity for menstrual blood, and the menstrual blood, as indicated by the menstrual blood 9' in Fig. 2(b), thinly spreads over the top sheet, thus allowing the contact area between the menstrual blood and top sheet to be increased. Presumably, a portion of the blood modifying agent 8 with the prescribed structure migrates into the menstrual blood during this time.

The present inventors have confirmed, through data for a triester of glycerin and fatty acid (PANACET 810s) which has higher hydrophobicity than the blood modifying agent of the present disclosure, that PANACET 810s reduces the viscosity of horse defibrinated blood by a maximum of about 90%, stably disperses erythrocytes in menstrual blood of healthy volunteers, prevents rouleaux, and lowers the surface tension of sheep defibrinated blood. The blood modifying agent specified in the claims of the present application also appears to have an effect similar to PANACET 810s.

Next, the menstrual blood 9', as indicated by the menstrual blood 9'' in Fig. 2(c), utilizes the size of the contact area for rapid permeation into the top sheet 2, and then as indicated by the menstrual blood 9''' in Fig. 2(d), it is completely drawn in by the action of the absorbent body 3.

For convenience of illustration in Fig. 2, a space is present between the top sheet 2 and the absorbent body 3, but in an actual absorbent article such a space will usually be absent. Therefore, the top sheet 2 having the prescribed thickness and basis weight will not easily retain menstrual blood in its interior, thereby allowing the absorbent body 3 to rapidly draw in the menstrual blood 9".

Because the blood modifying agent 8 has suitable affinity, and not excessive affinity, for menstrual blood, all of the menstrual blood can migrate into the absorbent body 3 without leaving menstrual blood on the top sheet 2, and especially on the skin contact surface 7 of the top sheet 2. As a result, the absorbent article of the present disclosure has no sticky feel on the top sheet and maintains a smooth top sheet, even after highly viscous menstrual blood has been absorbed. Furthermore, since menstrual blood does not easily remain on the skin contact surface of the top sheet, the skin contact surface of the top sheet is resistant to red coloration even after absorption of menstrual blood.

In the technical field, the skin contact surfaces of top sheets are coated with surfactants in order to alter the surface tension of blood and promote rapid absorption of blood. The difference in the mechanisms of the blood modifying agent described above and such surfactants will now be explained.

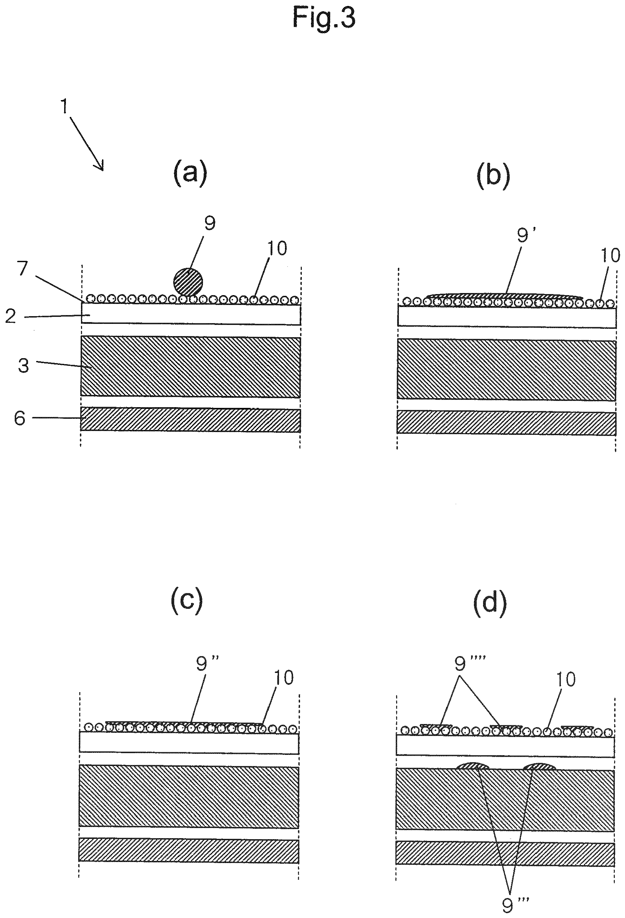

Fig. 3 is a schematic view showing the state of absorption of menstrual blood by an absorbent article having a surfactant coated on the skin contact surface of the top sheet. The sanitary napkin 1 shown in Fig. 3 comprises a liquid-permeable top sheet 2, a liquid-impermeable back sheet 6, and an absorbent body 3 between the liquid-permeable top sheet 2 and liquid-impermeable back sheet 6.

As shown in Fig. 3(a), highly viscous menstrual blood 9 reaches the skin contact surface 7 of the top sheet 2 and contacts with the surfactant 10. Because the surfactant 10 has very high affinity for menstrual blood 9 and lowers the surface tension of the menstrual blood 9, the menstrual blood 9' spreads across a wide region of the top sheet as shown in Fig. 3(b), and can increase its contact area with the top sheet. However, the top sheet 2 coated with the surfactant 10 has very high affinity for the hydrophilic components (blood plasma, etc.) in the menstrual blood 9'', and acts to attract them, causing the menstrual blood 9" to remain on the skin contact surface 7 of the top sheet 2. As a result, as shown in Fig. 3(d), although a portion of the menstrual blood 9''' permeates into the absorbent body 3, a portion of the menstrual blood 9'''' also remains on the skin contact surface 7 of the top sheet 2, leading to red coloration of the top sheet and a sticky feel.

Liquid-impermeable back sheets include films comprising PE and PP, air-permeable resin films, air-permeable resin films bonded to spunbond or spunlace nonwoven fabrics, and multilayer nonwoven fabrics such as SMS. In consideration of flexibility of the absorbent article, a low-density polyethylene (LDPE) film with a basis weight of about 15-30 g/m$^2$, for example, is preferred.

According to the absorbent article of the present disclosure, the absorbent article may comprise a second sheet between the liquid-permeable top sheet and the absorbent body. The second sheet may be any of the same nonwoven fabrics mentioned for the liquid-permeable top sheet.

A first example of the absorbent body is one having an absorbent core covered with a core wrap.

Examples of components for the absorbent core include hydrophilic fibers, including cellulose such as ground pulp or cotton, regenerated cellulose such as rayon or fibril rayon, semi-synthetic cellulose such as acetate or triacetate, particulate polymers, filamentous polymers, thermoplastic hydrophobic chemical fibers, and hydrophilicized thermoplastic hydrophobic chemical fibers, as well as combinations of the foregoing. The component of the absorbent core may also be a superabsorbent polymer, such as granules of a sodium acrylate copolymer or the like.

The core wrap is not particularly restricted so long as it is a substance that is liquid-permeable and with a barrier property that does not allow permeation of the polymer absorbent body, and it may be a woven fabric or nonwoven fabric, for example. The woven fabric or nonwoven fabric may be made of a natural fiber, chemical fiber, tissue, or the like.

A second example of the absorbent body is one formed from an absorbing sheet or polymer sheet, with a thickness of preferably about 0.3 to 5.0 mm. The absorbing sheet or polymer sheet may usually be used without any particular restrictions so long as it is one that can be used in an absorbent article such as a sanitary napkin.

As regards the region in the planar direction wherein the liquid-permeable top sheet includes a blood modifying agent, according to the absorbent article of the present disclosure, the liquid-permeable top sheet comprises a blood modifying agent in the excretory opening contact region. According to another absorbent article of the present disclosure, the liquid-permeable top sheet also comprises a blood modifying agent in regions other than the excretory opening contact region, in addition to the excretory opening contact region, and for example, it may comprise the blood modifying agent across the entire surface of the top sheet.

As regards the region in the thickness direction in which the liquid-permeable top sheet includes a blood modifying agent, according to the absorbent article of the present disclosure, the liquid-permeable top sheet comprises a blood modifying agent on the surface of the skin side, i.e. on the skin contact surface. If the blood modifying agent is present on the skin contact surface of the top sheet it will be possible for menstrual blood to thinly spread over the top sheet. According to another absorbent article of the present disclosure, the liquid-permeable top sheet is a nonwoven fabric, and it includes a blood modifying agent on the skin contact surface and in the interior between the skin contact surface and the clothing side surface. According to yet another absorbent article of the present disclosure, the liquid-permeable top sheet is a nonwoven fabric, and it includes a blood modifying agent over the entire thickness direction, i.e. on the skin contact surface, in the interior between the skin contact surface and the clothing side surface, and on the clothing side surface.

The liquid-permeable top sheet is formed from a nonwoven fabric and the blood modifying agent preferably does not fill the voids between the fibers of the nonwoven fabric and for example, the blood modifying agent may be attached as droplets or particulates on the surface of the nonwoven fabric fibers, or covering the surfaces of the fibers. The blood modifying agent also preferably has a large surface area, in order to rapidly act on menstrual blood that has reached the top sheet, and a blood modifying agent present as droplets or particulates preferably has a small droplet/particle size.

According to another absorbent article of the present disclosure, the absorbent article has a second sheet comprising a blood modifying agent. According to yet another absorbent article of the present disclosure, the absorbent article has an absorbent body comprising a blood modifying agent.

In this absorbent article, the top sheet comprises the blood modifying agent at a basis weight in the range of preferably between about 1 and about 30 g/m$^2$, more preferably between about 2 and about 20 g/m$^2$ and more preferably between about 3 and about 10 g/m$^2$. If the basis weight of the blood modifying agent is less than about 1 g/m$^2$ the amount of blood modifying agent may be insufficient, tending to result in residue of absorbed menstrual blood on the top sheet, and if the basis weight of the blood modifying agent is increased, the stickiness during wearing will tend to be increased.

There are no particular restrictions on the method of coating the blood modifying agent, and coating may be accomplished with heating as necessary, using a non-contact coater such as, for example, a spiral coater, curtain coater, spray coater or dip coater, or a contact coater or the like. A non-contact coater is preferred from the viewpoint of uniformly dispersing the droplet or particulate modifying agent throughout, and from the viewpoint of not causing damage in the material. The blood modifying agent may be coated directly, if it is a liquid at room temperature, or it may be heated to lower the viscosity, and when it is a solid at room temperature, it may be heated to liquefaction and coated through a control seam HMA gun. By increasing the air pressure of the control seam HMA gun, it is possible to coat the blood modifying agent as fine particulates.

The blood modifying agent may be coated during production of the top sheet material, i.e. the nonwoven fabric, or it may be coated in the manufacturing line for production of the absorbent article. From the viewpoint of minimizing equipment investment, the blood modifying agent is preferably coated in the manufacturing line for the absorbent article, and in order to prevent shedding of the blood modifying agent which may contaminate the line, the blood modifying agent is preferably coated during a step downstream from the manufacturing line, and specifically, immediately before encapsulation of the product in an individual package.

The blood modifying agent also has an effect as a lubricant. Since the top sheet is a nonwoven fabric, the blood modifying agent reduces friction between the fibers composing the top sheet, and can improve flexibility across the entire nonwoven fabric.

The absorbent article is one that is intended for absorption of blood, such as a sanitary napkin or panty liner.

An absorbent article of the present disclosure does not require components such as emollients and immobilizing agents, unlike in an absorbent article containing a conventionally known skin care composition, lotion composition or the like, and the blood modifying agent alone may be applied to the top sheet.

Examples

The present disclosure will now be explained by examples, with the understanding that it is not meant to be limited to the examples.

[Example 1]

[Measurement of blood contact angle and blood residue rate]

The blood modifying agent was evaluated in terms of the blood contact angle and the blood residue rate. The samples and evaluation method used in the test were as follows.

[Blood modifying agent types]

- UNILUBE MS-70K, product of NOF Corp.
  PPG-15 Stearyl ether, weight-average molecular weight: approximately 1,140
- UNIOL D-400, product of NOF Corp.
  Polyoxypropylene glycol, weight-average molecular weight: approximately 400
- UNIOL D-1000, product of NOF Corp.
  Polyoxypropylene glycol, weight-average molecular weight: approximately 1,000
- PANACET 810s, product of NOF Corp.
  Glycerin and fatty acid triester with $C_8$ fatty acid:$C_{10}$ fatty acid at a mass ratio of about 85:15, weight-average molecular weight: approximately 480
- PARLEAM 6, product of NOF Corp.
  Branched chain hydrocarbon, produced by copolymerization of liquid isoparaffin, isobutene and n-butene followed by hydrogen addition, polymerization degree: approximately 5-10, weight-average molecular weight: approximately 330

[Nonwoven fabric type]

- Spunbond (SB), product of Asahi Kasei Corp.
  ELTAS, thickness: 0.22 mm, basis weight: 20.8 g/m$^2$
- Spunbond/meltblown/spunbond (SMS), product of Mitsui Chemicals, Inc.
  TPQ, thickness: 0.12 mm, basis weight: 13.5 g/m$^2$
- Air-through (AT), product of Unicharm
  Fiber size: 2.2 dtex, thickness: 0.93 mm, basis weight: 27.2 g/m$^2$

[Blood contact angle measurement method]

1. A polyethylene film is anchored on an acrylic board, and a pipette and scraper are used to coat the blood modifying agent on the PE film to a basis weight of approximately 10 g/m$^2$.
2. One drop (approximately 0.1 mL) of horse defibrinated blood (Nihon Bio-Supp Center) is added to the blood modifying agent through a pipette, and the contact angle is measured.
3. The contact angle is measured from a cross-sectional image of the droplet of the horse defibrinated blood.

The contact angle is measured in a thermostatic chamber at 20°C.
A polyethylene film is used because it is a common material for top sheets.

[Blood residue rate measurement method]

1. The blood modifying agent is coated on the nonwoven fabric using a control seam HMA gun, at room temperature. The basis weights for SB, SMS and AT are approximately 10, 15 and 15 g/m$^2$, respectively. The initial weight ($W_{N0}$) of the nonwoven fabric is then measured.
2. Five weight-measured filter sheets ($W_{P0}$) are placed on the acrylic board, and the nonwoven fabric is situated thereover, with the blood modifying agent-coated side facing upward.
3. One drop (approximately 0.1 mL) of horse defibrinated blood (Nihon Bio-Supp Center) is added to the blood modifying agent-coated side of the nonwoven fabric through a pipette.
4. Approximately 10 seconds after dropping of the horse defibrinated blood, another drop (approximately 0.1 mL) of horse defibrinated blood is dropped onto a different location of the blood modifying agent-coated side, and after about 1 second, the acrylic board is inclined 15°.
5. One minute after the second dropping, the post-test weight of the nonwoven fabric ($W_{N1}$) and the post-test weight of the filter sheet ($W_{P1}$) are measured, and the blood residue rate is calculated by the following formula.

$$\text{Blood residue rate (\%)} = 100 \times (W_{N1} - W_{N0}) / [(W_{N1} - W_{N0}) + (W_{P1} - W_{P0})]$$

The acrylic board is inclined 15° in order to minimize the effect of gravity, and to evaluate the effect of the blood modifying agent and the drawing force of the filter sheet (corresponding to an absorbent body) on the horse defibrinated blood.
The blood residue rate is measured in a thermostatic chamber at 20°C.
The measurement results for the blood contact angle and the blood residue rate are summarized in Table 1 below, together with the properties of the blood modifying agents.

[Table 1]

| No. | Blood modifying agent | Water holding percentage (%) | Contact angle (°) | Blood residue rate (%) | | |
|-----|----------------------|------------------------------|-------------------|------|------|------|
| | | | | SB | SMS | TA |
| 1-1 | MS-70K | 2.8 | 10 | 10 | 1 | 20 |
| 1-2 | D-400 | 26.7<< | 0 | 29 | 7 | 71 |
| 1-3 | D-1000 | 26.7 | 0 | 27 | 8 | 50 |
| 1-4 | PANACET 810s | 0.3 | 20 | 100 | 100 | 100 |
| 1-5 | PARLEAM 6 | 0.06 | 20 | 100 | 100 | 100 |
| 1-6 | - | - | 75 | | | |

Table 1 shows that Nos. 1-1 to 1-3 had small contact angles, and low blood residue rates on each nonwoven fabric, while Nos. 1-4 and 1-5 had large contact angles and high blood residue rates. This suggests that with Nos. 1-4 and 1-5, spread of blood was more difficult, impeding its penetration into the nonwoven fabrics, and that the filter sheets did not easily draw up blood.

[Example 2]

[Absorbent article production and test]

There were prepared the SB described in Example 1 as a top sheet, a second sheet, formed of an air-through nonwoven fabric (composite fiber composed of polyester and polyethylene terephthalate, basis weight: 30 g/m$^2$), an absorbent body comprising pulp (basis weight: 150-450 g/m$^2$, increased at the center section), an acrylic superabsorbent polymer (basis weight: 15 g/m$^2$) and tissue as a core wrap, a water-repellent agent-treated side sheet, and a back sheet composed of a polyethylene film.

Using the five different compounds described in Example 1 as blood modifying agents, each was coated onto the skin contact surface of the top sheet from a control seam HMA gun at room temperature, to a basis weight of 5.0 g/m$^2$. The blood modifying agent was coated over roughly the entire skin contact surface of the top sheet.

Next, a back sheet, an absorbent body, a second sheet, and a top sheet with the blood modifying agent-coated side facing upward, were stacked in that order to form five sanitary napkins with different blood modifying agents. For reference there was also prepared one absorbent article not coated with a blood modifying agent, for a total of 6 absorbent articles.

The top sheet was changed from SB to SMS or TA to produce sanitary napkins (2 types × 6 sheets), for a total of 3 different types × 6 sanitary napkins.

This procedure was repeated, using several samples with different top sheets and blood modifying agents, to prepare several different of each.

Subjects were asked to wear the samples, yielding essentially the same results as the data for the blood residue rate, and the responses indicated that after absorption of menstrual blood, the top sheet was smoother with MS-70K than with the others. The responses also indicated a high smooth feel with SMS and SB, and especially with SMS.

The present disclosure relates to the following J1 to J12. [J1]

An absorbent article comprising a liquid-permeable top sheet, a liquid-impermeable back sheet and an absorbent body between the liquid-permeable top sheet and the liquid-impermeable back sheet,

wherein the liquid-permeable top sheet has a thickness of 0.10 to 0.30 mm and a basis weight of 5 to 30 g/m2 in the excretory opening contact region,

the liquid-permeable top sheet includes a blood modifying agent on the skin contact surface of the excretory opening contact region, and

the blood modifying agent is a polyoxypropylene glycol-based compound.

[J2]

The absorbent article according to J1, wherein the liquid-permeable top sheet is a spunbond nonwoven fabric or an SMS nonwoven fabric.

[J3]

The absorbent article according to J1, wherein the liquid-permeable top sheet is a porous film.

[J4]

The absorbent article according to any one of J1 to J3, wherein the blood modifying agent is selected from the group consisting of the following (A) to (E), and any combination thereof:

(A) polyoxypropylene glycol,
(B) a mono or diether of (B1) polyoxypropylene glycol and (B2) a compound having a hydrocarbon moiety and one hydroxyl group substituting for a hydrogen of the hydrocarbon moiety,
(C) a mono or diester of (C1) polyoxypropylene glycol and (C2) a compound having a hydrocarbon moiety and one carboxyl group substituting for a hydrogen of the hydrocarbon moiety,
(D) a compound produced by addition of (D2) one or more propylene oxides to (D1) a compound having a hydrocarbon moiety and 2-6 hydroxyl groups substituting for hydrogens of the hydrocarbon moiety or dehydrating condensate thereof, and optional esterification with (D3) a compound having a hydrocarbon moiety and one carboxyl group substituting for a hydrogen of the hydrocarbon moiety or (D4) phosphoric acid, and

(E) a compound produced by addition of (E2) one or more propylene oxides to (E1) an aliphatic amide, aliphatic amine or alkylglucose, and optional esterification with (E3) a compound having a hydrocarbon moiety and one carboxyl group substituting for a hydrogen of the hydrocarbon moiety or (E4) phosphoric acid.

[J5]

The absorbent article according to J4, wherein the (B2) compound having a hydrocarbon moiety and one hydroxyl group substituting for a hydrogen of the hydrocarbon moiety is a C1-C20 aliphatic monohydric alcohol.

[J6]

The absorbent article according to J4 or J5, wherein the (C2) compound having a hydrocarbon moiety and one carboxyl group substituting for a hydrogen of the hydrocarbon moiety is a C2-C30 fatty acid.

[J7]

The absorbent article according to any one of J4 to J6, wherein the (D1) compound having a hydrocarbon moiety and 2-6 hydroxyl groups substituting for hydrogens of the hydrocarbon moiety or dehydrating condensate thereof, is selected from the group consisting of chain hydrocarbon hexaols, chain hydrocarbon pentaols, chain hydrocarbon tetraols, chain hydrocarbon triols and chain hydrocarbon diols, and dehydrating condensates thereof.

[J8]

The absorbent article according to any one of J4 to J7, wherein the (D1) compound having a hydrocarbon moiety and 2-6 hydroxyl groups substituting for hydrogens of the hydrocarbon moiety or dehydrating condensate thereof, is selected from the group consisting of sorbitol, pentaerythritol, glycerin, trimethylolpropane and ethylene glycol, and dehydrating condensates thereof.

[J9]

The absorbent article according to any one of J4 to J8, wherein the (D3) compound having a hydrocarbon moiety and one carboxyl group substituting for a hydrogen of the hydrocarbon moiety or the (E3) compound having a hydrocarbon moiety and one carboxyl group substituting for a hydrogen of the hydrocarbon moiety is a C2-C30 fatty acid.

[J10]

The absorbent article according to any one of J4 to J9, wherein the (E1) aliphatic amide, aliphatic amine or alkylglucose is a C1-C20 alkylamide, C1-C20 alkylamine or methylglucose, respectively.

[J11]

The absorbent article according to any one of J1 to J10, wherein the blood modifying agent is selected from the group consisting of PPG-4 butyl ether, PPG-12 butyl ether, PPG-17 butyl ether, PPG-20 butyl ether, PPG-24 butyl ether, PPG-33 butyl ether, PPG-40 butyl ether, PPG-52 butyl ether, PPG-3 myristyl ether, PPG-10 cetyl ether, PPG-11 stearyl ether, PPG-15 stearyl ether, PPG-2 lanolyl ether, PPG-5 lanolyl ether, PPG-10 lanolyl ether, PPG-20 lanolyl ether, PPG-26 oleate, PPG-36 oleate, PPG-5.5 castorate, PPG-6 glyceryl ether, PPG-8 glyceryl ether, PPG-10 glyceryl ether, PPG-16 glyceryl ether, PPG-9 diglyceryl ether, PPG-14 diglyceryl ether, PPG-25 sorbitol and PPG-33 sorbitol, and any desired combinations of the foregoing.

[J12]

The absorbent article according to any one of J1 to J11, which is a sanitary napkin or panty liner.

[References Signs List]

1 Sanitary napkin

2 Top sheet
3 Absorbent body
4 Side sheet
5 Stamped section
6 Back sheet
7 Skin contact surface
8 Blood modifying agent
9 Menstrual blood
10 Surfactant

**Claims**

1. The use of an agent in a liquid-permeable top sheet (2) in an absorbent article (1) for modifying blood on the liquid-permeable top sheet,
   wherein the absorbent article comprises the liquid-permeable top sheet (2), a liquid-impermeable back sheet (6) and an absorbent body (3) between the liquid-permeable top sheet and the liquid-impermeable back sheet,

   wherein the liquid-permeable top sheet (2) is a spunbond nonwoven fabric or an SMS nonwoven fabric and has a thickness of 0.10 to 0.30 mm and a basis weight of 5 to 30 $g/m^2$ in the excretory opening contact region, the liquid-permeable top sheet includes a blood modifying agent (8) on the skin contact surface (7) of the excretory opening contact region, and
   the blood modifying agent (8) is a polyoxypropylene glycol-based compound having a water holding percentage of 1.5 to 40.0 mass% measured as described in the description.

2. The use of an agent according to claim 1, wherein the blood modifying agent (8) is selected from the group consisting of the following (A) to (E), and any combination thereof:

   (A) polyoxypropylene glycol,
   (B) a mono or diether of (B1) polyoxypropylene glycol and (B2) a compound having a hydrocarbon moiety and one hydroxyl group substituting for a hydrogen of the hydrocarbon moiety,
   (C) a mono or diester of (C1) polyoxypropylene glycol and (C2) a compound having a hydrocarbon moiety and one carboxyl group substituting for a hydrogen of the hydrocarbon moiety,
   (D) a compound produced by addition of (D2) one or more propylene oxides to (D1) a compound having a hydrocarbon moiety and 2-6 hydroxyl groups substituting for hydrogens of the hydrocarbon moiety or dehydrating condensate thereof, and
   (E) a compound produced by addition of (E2) one or more propylene oxides to (E1) an aliphatic amide, aliphatic amine or alkylglucose.

3. The use of an agent according to claim 1, wherein the blood modifying agent (8) is selected from the group consisting of the following (A) to (E), and any combination thereof:

   (A) polyoxypropylene glycol,
   (B) a mono or diether of (B1) polyoxypropylene glycol and (B2) a compound having a hydrocarbon moiety and one hydroxyl group substituting for a hydrogen of the hydrocarbon moiety,
   (C) a mono or diester of (C1) polyoxypropylene glycol and (C2) a compound having a hydrocarbon moiety and one carboxyl group substituting for a hydrogen of the hydrocarbon moiety,
   (D) a compound produced by addition of (D2) one or more propylene oxides to (D1) a compound having a hydrocarbon moiety and 2-6 hydroxyl groups substituting for hydrogens of the hydrocarbon moiety or dehydrating condensate thereof, and esterification with (D3) a compound having a hydrocarbon moiety and one carboxyl group substituting for a hydrogen of the hydrocarbon moiety or (D4) phosphoric acid, and
   (E) a compound produced by addition of (E2) one or more propylene oxides to (E1) an aliphatic amide, aliphatic amine or alkylglucose, and esterification with (E3) a compound having a hydrocarbon moiety and one carboxyl group substituting for a hydrogen of the hydrocarbon moiety or (E4) phosphoric acid.

4. The use of an agent according to claim 2 or 3, wherein the (B2) compound having a hydrocarbon moiety and one hydroxyl group substituting for a hydrogen of the hydrocarbon moiety is a $C_1$-$C_{20}$ aliphatic monohydric alcohol.

**5.** The use of an agent according to any one of claims 2 to 4, wherein the (C2) compound having a hydrocarbon moiety and one carboxyl group substituting for a hydrogen of the hydrocarbon moiety is a $C_2$-$C_{30}$ fatty acid.

**6.** The use of an agent according to any one of claims 2 to 5, wherein the (D1) compound having a hydrocarbon moiety and 2-6 hydroxyl groups substituting for hydrogens of the hydrocarbon moiety or dehydrating condensate thereof, is selected from the group consisting of chain hydrocarbon hexaols, chain hydrocarbon pentaols, chain hydrocarbon tetraols, chain hydrocarbon triols and chain hydrocarbon diols, and dehydrating condensates thereof.

**7.** The use of an agent according to any one of claims 2 to 6, wherein the (D1) compound having a hydrocarbon moiety and 2-6 hydroxyl groups substituting for hydrogens of the hydrocarbon moiety or dehydrating condensate thereof, is selected from the group consisting of sorbitol, pentaerythritol, glycerin, trimethylolpropane and ethylene glycol, and dehydrating condensates thereof.

**8.** The use of an agent according to any one of claims 2 to 7, wherein the (D3) compound having a hydrocarbon moiety and one carboxyl group substituting for a hydrogen of the hydrocarbon moiety or the (E3) compound having a hydrocarbon moiety and one carboxyl group substituting for a hydrogen of the hydrocarbon moiety is a $C_2$-$C_{30}$ fatty acid.

**9.** The use of an agent according to any one of claims 2 to 8, wherein the (E1) aliphatic amide, aliphatic amine or alkylglucose is a $C_1$-$C_{20}$ alkylamide, $C_1$-$C_{20}$ alkylamine or methylglucose, respectively.

**10.** The use of an agent according to any one of claims 1 to 9, wherein the blood modifying agent is selected from the group consisting of PPG-4 butyl ether, PPG-12 butyl ether, PPG-17 butyl ether, PPG-20 butyl ether, PPG-24 butyl ether, PPG-33 butyl ether, PPG-40 butyl ether, PPG-52 butyl ether, PPG-3 myristyl ether, PPG-10 cetyl ether, PPG-11 stearyl ether, PPG-15 stearyl ether, PPG-2 lanolyl ether, PPG-5 lanolyl ether, PPG-10 lanolyl ether, PPG-20 lanolyl ether, PPG-26 oleate, PPG-36 oleate, PPG-5.5 castorate, PPG-6 glyceryl ether, PPG-8 glyceryl ether, PPG-10 glyceryl ether, PPG-16 glyceryl ether, PPG-9 diglyceryl ether, PPG-14 diglyceryl ether, PPG-25 sorbitol and PPG-33 sorbitol, and any desired combinations of the foregoing.

**11.** The use of an agent according to any one of claims 1 to 10, wherein the absorbent article is is a sanitary napkin or panty liner.

**Patentansprüche**

**1.** Verwendung eines Mittels in einer flüssigkeitsdurchlässigen oberen Lage (2) in einem absorbierenden Artikel (1) für die Modifizierung von Blut auf der flüssigkeitsdurchlässigen oberen Lage,
wobei der absorbierende Artikel Folgendes umfasst: die flüssigkeitsdurchlässige obere Lage (2), eine flüssigkeitsundurchlässige hintere Lage (6) und einen Saugkörper (3) zwischen der flüssigkeitsdurchlässigen oberen Lage und der flüssigkeitsundurchlässigen hinteren Lage,

wobei die flüssigkeitsdurchlässige obere Lage (2) ein Spinnvliesstoff oder ein SMS-Vliesstoff ist und eine Dicke von 0,10 bis 0,30 mm und ein Flächengewicht von 5 bis 30 g/m$^2$ in dem Ausscheidungsöffnungskontaktbereich aufweist,
die flüssigkeitsdurchlässige obere Lage ein Blutmodifizierungsmittel (8) auf der Hautkontaktoberfläche (7) des Ausscheidungsöffnungskontaktbereichs einschließt und
das Blutmodifizierungsmittel (8) eine Polyoxypropylenglycol-basierte Verbindung ist, die einen Wasseraufnahme-Prozentsatz von 1,5 bis 40,0 Masse-%, gemessen wie in der Beschreibung beschrieben, aufweist.

**2.** Verwendung eines Mittels nach Anspruch 1, wobei das Blutmodifizierungsmittel (8) aus der Gruppe, die aus den folgenden (A) bis (E) besteht, und jedweder Kombination davon ausgewählt ist:

(A) Polyoxypropylenglycol,
(B) einem Mono- oder Diether von (B1) Polyoxypropylenglycol und (B2) einer Verbindung, die eine Kohlenwasserstoffeinheit und eine Hydroxylgruppe, die einen Wasserstoff der Kohlenwasserstoffeinheit substituiert, aufweist;
(C) einem Mono- oder Diester von (C1) Polyoxypropylenglycol und (C2) einer Verbindung, die eine Kohlenwasserstoffeinheit und eine Carboxylgruppe, die einen Wasserstoff der Kohlenwasserstoffeinheit substituiert, aufweist;

(D) einer Verbindung, die durch Folgendes erzeugt wird: Addition von (D2) einem oder mehreren Propylenoxiden an (D1) eine Verbindung, die eine Kohlenwasserstoffeinheit und 2-6 Hydroxylgruppen, die Wasserstoffe der Kohlenwasserstoffeinheit substituieren, aufweist, oder ein Dehydratisierungskondensat davon, und

(E) einer Verbindung, die durch Folgendes erzeugt wird: Addition von (E2) einem oder mehreren Propylenoxiden an (E1) ein aliphatisches Amid, aliphatisches Amin oder Alkylglucose.

3. Verwendung eines Mittels nach Anspruch 1, wobei das Blutmodifizierungsmittel (8) aus der Gruppe, die aus den folgenden (A) bis (E) besteht, und jedweder Kombination davon ausgewählt ist:

(A) Polyoxypropylenglycol,

(B) einem Mono- oder Diether von (B1) Polyoxypropylenglycol und (B2) einer Verbindung, die eine Kohlenwasserstoffeinheit und eine Hydroxylgruppe, die einen Wasserstoff der Kohlenwasserstoffeinheit substituiert, aufweist;

(C) einem Mono- oder Diester von (C1) Polyoxypropylenglycol und (C2) einer Verbindung, die eine Kohlenwasserstoffeinheit und eine Carboxylgruppe, die einen Wasserstoff der Kohlenwasserstoffeinheit substituiert, aufweist;

(D) einer Verbindung, die durch Folgendes erzeugt wird: Addition von (D2) einem oder mehreren Propylenoxiden an (D1) eine Verbindung, die eine Kohlenwasserstoffeinheit und 2-6 Hydroxylgruppen, die Wasserstoffe der Kohlenwasserstoffeinheit substituieren, aufweist, oder ein Dehydratisierungskondensat davon, und Veresterung mit (D3) einer Verbindung, die eine Kohlenwasserstoffeinheit und eine Carboxylgruppe, die einen Wasserstoff der Kohlenwasserstoffeinheit substituiert, aufweist, oder (D4) Phosphorsäure, und

(E) einer Verbindung, die durch Folgendes erzeugt wird: Addition von (E2) einem oder mehreren Propylenoxiden an (E1) ein aliphatisches Amid, aliphatisches Amin oder Alkylglucose und Veresterung mit (E3) einer Verbindung, die eine Kohlenwasserstoffeinheit und eine Carboxylgruppe, die einen Wasserstoff der Kohlenwasserstoffeinheit substituiert, aufweist, oder (E4) Phosphorsäure.

4. Verwendung eines Mittels nach Anspruch 2 oder 3, wobei die (B2) Verbindung, die eine Kohlenwasserstoffeinheit und eine Hydroxylgruppe, die einen Wasserstoff der Kohlenwasserstoffeinheit substituiert, aufweist, ein einwertiger aliphatischer $C_1$-$C_{20}$-Alkohol ist.

5. Verwendung eines Mittels nach einem der Ansprüche 2 bis 4, wobei die (C2) Verbindung, die eine Kohlenwasserstoffeinheit und eine Carboxylgruppe, die einen Wasserstoff der Kohlenwasserstoffeinheit substituiert, aufweist, eine $C_2$-$C_{30}$-Fettsäure ist.

6. Verwendung eines Mittels nach einem der Ansprüche 2 bis 5, wobei die (D1) Verbindung, die eine Kohlenwasserstoffeinheit und 2-6 Hydroxylgruppen, die Wasserstoffe der Kohlenwasserstoffeinheit substituieren, aufweist, oder ein Dehydratisierungskondensat davon aus der Gruppe ausgewählt ist, die aus Folgenden besteht: Kettenkohlenwasserstoffhexaolen, Kettenkohlenwasserstoffpentaolen, Kettenkohlenwasserstofftetraolen, Kettenkohlenwasserstofftriolen und Kettenkohlenwasserstoffdiolen, und Dehydratisierungskondensaten davon.

7. Verwendung eines Mittels nach einem der Ansprüche 2 bis 6, wobei die (D1) Verbindung, die eine Kohlenwasserstoffeinheit und 2-6 Hydroxylgruppen, die Wasserstoffe der Kohlenwasserstoffeinheit substituieren, aufweist, oder ein Dehydratisierungskondensat davon aus der Gruppe ausgewählt ist, die aus Folgenden besteht: Sorbitol, Pentaerythritol, Glycerin, Trimethylolpropan und Ethylenglycol, und Dehydratisierungskondensaten davon.

8. Verwendung eines Mittels nach einem der Ansprüche 2 bis 7, wobei die (D3) Verbindung, die eine Kohlenwasserstoffeinheit und eine Carboxylgruppe, die einen Wasserstoff der Kohlenwasserstoffeinheit substituiert, aufweist, oder die (E3) Verbindung, die eine Kohlenwasserstoffeinheit und eine Carboxylgruppe, die einen Wasserstoff der Kohlenwasserstoffeinheit substituiert, aufweist, eine $C_2$-$C_{30}$-Fettsäure ist.

9. Verwendung eines Mittels nach einem der Ansprüche 2 bis 8, wobei das (E1) aliphatische Amid, aliphatische Amin oder Alkylglucose ein $C_1$-$C_{20}$-Alkylamid, $C_1$-$C_{20}$-Alkylamin bzw. Methylglucose ist.

10. Verwendung eines Mittels nach einem der Ansprüche 1 bis 9, wobei das Blutmodifizierungsmittel aus der Gruppe ausgewählt ist, die aus Folgenden besteht: PPG-4-Butylether, PPG-12-Butylether, PPG-17-Butylether, PPG-20-Butylether, PPG-24-Butylether, PPG-33-Butylether, PPG-40-Butylether, PPG-52-Butylether, PPG-3-Myristylether, PPG-10-Cetylether, PPG-11-Stearylether, PPG-15-Stearylether, PPG-2-Lanolylether, PPG-5-Lanolylether, PPG-10-Lanolylether, PPG-20-Lanolylether, PPG-26-Oleat, PPG-36-Oleat, PPG-5.5-Castorat, PPG-6-Glycerylether, PPG-8-Glycerylether, PPG-10-Glycerylether, PPG-16-Glycerylether, PPG-9-Diglycerylether, PPG-14-Diglycerylether, PPG-25-Sorbitol und PPG-33-Sorbitol, und jedweden gewünschten Kombinationen der Vorstehenden.

**11.** Verwendung eines Mittels nach einem der Ansprüche 1 bis 10, wobei der absorbierende Artikel eine Monatsbinde oder Slipeinlage ist.

**Revendications**

**1.** Utilisation d'un agent dans une feuille supérieure perméable aux liquides (2) d'un article absorbant (1) pour modifier le sang sur la feuille supérieure perméable aux liquides,
où l'article absorbant comprend la feuille supérieure perméable aux liquides (2), une feuille de support imperméable aux liquides (6) et un noyau absorbant (3) entre la feuille supérieure perméable aux liquides et la feuille de support imperméable aux liquides,
où la feuille supérieure perméable aux liquides (2) est un tissu non tissé obtenu par filage-nappage ou un tissu non tissé obtenu par filage-nappage/extrusion-soufflage/filage nappage ayant une épaisseur qui va de 0,10 à 0,30 mm et une masse surfacique qui va de 5 à 30 g/m$^2$ dans la région en contact avec l'orifice d'excrétion,
la feuille supérieure perméable aux liquides inclut un agent modifiant le sang (8) sur la surface en contact avec la peau (7) de la région en contact avec l'orifice d'excrétion et
l'agent modifiant le sang (8) est un composé à base d'un poly(oxypropylène glycol) ayant un pourcentage de capacité de rétention de l'eau, mesuré comme décrit dans la description, qui va de 1,5 à 40,0 % en masse.

**2.** Utilisation d'un agent selon la revendication 1, où l'agent modifiant le sang (8) est sélectionné dans le groupe consistant en les composés (A)-(E) suivants et toute combinaison de ceux-ci :

(A) un poly(oxypropylène glycol),
(B) un mono ou un diéther d'un poly(oxypropylène glycol) (B1) et d'un composé ayant un fragment hydrocarboné et un groupement hydroxyle substituant un hydrogène du fragment hydrocarboné (B2),
(C) un mono ou un diester d'un poly(oxypropylène glycol) (C1) et d'un composé ayant un fragment hydrocarboné et un groupement carboxyle substituant un hydrogène du fragment hydrocarboné (C2),
(D) un composé produit par addition de un ou plusieurs oxydes de propylène (D2) à un composé ayant un fragment hydrocarboné et 2-6 groupements hydroxyles substituant des hydrogènes du fragment hydrocarboné (D1) ou un condensat de déshydratation de celui-ci et
(E) un composé produit par addition de un ou plusieurs oxydes de propylène (E2) à un amide aliphatique, une amine aliphatique ou un alkylglucose (E1).

**3.** Utilisation d'un agent selon la revendication 1, où l'agent modifiant le sang (8) est sélectionné dans le groupe consistant en les composés (A)-(E) suivants et toute combinaison de ceux-ci :

(A) un poly(oxypropylène glycol),
(B) un mono ou un diéther d'un poly(oxypropylène glycol) (B1) et d'un composé ayant un fragment hydrocarboné et un groupement hydroxyle substituant un hydrogène du fragment hydrocarboné (B2),
(C) un mono ou un diester d'un poly(oxypropylène glycol) (C1) et d'un composé ayant un fragment hydrocarboné et un groupement carboxyle substituant un hydrogène du fragment hydrocarboné (C2),
(D) un composé produit par addition de un ou plusieurs oxydes de propylène (D2) à un composé ayant un fragment hydrocarboné et 2-6 groupements hydroxyles substituant des hydrogènes du fragment hydrocarboné (D1) ou un condensat de déshydratation de celui-ci et estérification avec un composé ayant un fragment hydrocarboné et un groupement carboxyle substituant un hydrogène du fragment hydrocarboné (D3) ou un acide phosphorique (D4) et
(E) un composé produit par addition de un ou plusieurs oxydes de propylène (E2) à un amide aliphatique, une amine aliphatique ou un alkylglucose (E1) et estérification avec un composé ayant un fragment hydrocarboné et un groupement carboxyle substituant un hydrogène du fragment hydrocarboné (E3) ou un acide phosphorique (E4).

**4.** Utilisation d'un agent selon la revendication 2 ou 3, où le composé ayant un fragment hydrocarboné et un groupement hydroxyle substituant un hydrogène du fragment hydrocarboné (B2) est un alcool monohydrique aliphatique en $C_1$-$C_{20}$.

5.  Utilisation d'un agent selon l'une quelconque des revendications 2 à 4, où le composé ayant un fragment hydrocarboné et un groupement carboxyle substituant un hydrogène du fragment hydrocarboné (C2) est un acide gras en $C_2$-$C_{30}$.

6.  Utilisation d'un agent selon l'une quelconque des revendications 2 à 5, où le composé ayant un fragment hydrocarboné et 2-6 groupements hydroxyles substituant des hydrogènes du fragment hydrocarboné (D1) ou un condensat de déshydratation de celui-ci est sélectionné dans le groupe consistant en des hexaols à chaîne hydrocarbonée, des pentaols à chaîne hydrocarbonée, des tétraols à chaîne hydrocarbonée, des triols à chaîne hydrocarbonée et des diols à chaîne hydrocarbonée et des condensats de déshydratation de ceux-ci.

7.  Utilisation d'un agent selon l'une quelconque des revendications 2 à 6, où le composé ayant un fragment hydrocarboné et 2-6 groupements hydroxyles substituant des hydrogènes du fragment hydrocarboné (D1) ou un condensat de déshydratation de celui-ci est sélectionné dans le groupe consistant en le sorbitol, le pentaérythritol, la glycérine, le triméthylolpropane et l'éthylène glycol et des condensats de déshydratation de ceux-ci.

8.  Utilisation d'un agent selon l'une quelconque des revendications 2 à 7, où le composé ayant un fragment hydrocarboné et un groupement carboxyle substituant un hydrogène du fragment hydrocarboné (D3) ou le composé ayant un fragment hydrocarboné et un groupement carboxyle substituant un hydrogène du fragment hydrocarboné (E3) est un acide gras en $C_2$-$C_{30}$.

9.  Utilisation d'un agent selon l'une quelconque des revendications 2 à 8, où l'amide aliphatique, l'amine aliphatique ou l'alkylglucose (E1) est un alkylamide en $C_1$-$C_{20}$, une alkylamine en $C_1$-$C_{20}$ ou le méthylglucose, respectivement.

10. Utilisation d'un agent selon l'une quelconque des revendications 1 à 9, où l'agent modifiant le sang est sélectionné dans le groupe consistant en les suivants : éther butylique de PPG-4, éther butylique de PPG-12, éther butylique de PPG-17, éther butylique de PPG-20, éther butylique de PPG-24, éther butylique de PPG-33, éther butylique de PPG-40, éther butylique de PPG-52, éther myristylique de PPG-3, éther cétylique de PPG-10, éther stéarylique de PPG-11, éther stéarylique de PPG-15, éther lanolylique de PPG-2, éther lanolylique de PPG-5, éther lanolylique de PPG-10, éther lanolylique de PPG-20, oléate de PPG-26, oléate de PPG-36, castorate de PPG-5.5, éther glycérylique de PPG-6, éther glycérylique de PPG-8, éther glycérylique de PPG-10, éther glycérylique de PPG-16, éther diglycérylique de PPG-9, éther diglycérylique de PPG-14, sorbitol PPG-25 et sorbitol PPG-33 et toute combinaison désirée de ceux-ci.

11. Utilisation d'un agent selon l'une quelconque des revendications 1 à 10, où l'article absorbant est une serviette périodique ou un protège-slip.

# Fig.1

# Fig.2

# Fig.3

**EP 2 821 039 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010518918 A
- JP 2011510801 A
- US 20090209930 A1
- US 20110070277 A1
- WO 03028776 A1